# EUROPEAN PATENT APPLICATION

(11) **EP 1 359 131 A1**
(43) Date of publication of application: **05.11.2003**
(21) Application number: 02447076.7
(22) Date of filing: 26.04.2002
(51) Int. Cl.: C04B 35/634, C04B 38/00, B22F 1/00

(54) **Method for producing metallic and ceramic products**

(71) Applicant: "VLAAMSE INSTELLING VOOR TECHNOLOGISCH ONDERZOEK", afgekort "V.I.T.O.", 2400 Mol (BE)
(72) Inventor: Cooymans, Jozef, 2400 Mol (NL); De Wilde, Anne-Marie, 2400 Mol (BE); Thijs, Ivo, 2400 Mol (NE); Mullens, Steven, 3740 Bilzen (BE); Snijkers, Frans, 3930 Hamont-Achel (BE); Luyten, Jan, 3294 Diest (BE)
(74) Representative: Vandersteen, Pieter

(57) **Abstract**

The present invention is related to a method for producing metallic or ceramic products, comprising the steps of:
- Providing a suitable ceramic or metallic slurry,
- Casting said slurry into a predefined form,
- Drying, optionally calcining and sintering said form,
characterised in that said suitable ceramic or metallic slurry comprises a biogel former.

## Description

### Field of the invention

The present invention is related to a method for producing metallic and ceramic products. More particularly, the present invention is related to the use of natural gel forming compounds in a method for producing metallic or ceramic products, advantageously for tape casting and for the production of foams. The present invention is further related to a method for producing metallic or ceramic foams having a controlled cell structure using hollow shapes.

### State of the art

Ceramic products can be manufactured by conventional (dry) techniques on one hand, like pressing (uni-axial or hot or cold isostatical) or, on the other hand, by more advanced (wet) techniques. Such techniques that use a suspension are tape casting and slurry coating. The latter processing route requires that the ceramic powder is part of a suspension in an intermediate stage of the process. Further processing usually consists of drying, calcining and sintering. In addition, machining of the 'green' or the sintered product is possible.

The suspension used for this tape casting technique consists typically of a ceramic powder, a dispersing medium, a dispersant and a binder. In case of tape casting usually at least one or a mixture of two plasticisers are added. Optionally, wetting agents surfactants and antifoaming agents can be added. The dispersing medium can be either an organic solvent or water. The binder is typically a synthetic organic compound, which is often harmful for the environment.

In general a tape casting process is done according to the following process sequence: Suspension preparation/de-gassing, tapecasting, drying, removal from the substrate, calcination and sintering. Ceramic products obtained in this way are used for various purposes. So is tape casting a well-known technique to produce ceramic substrates of various chemical compounds like alumina (Al₂O₃), aluminium nitride (AlN) and mullite (3Al₂O₃.2SiO₂) that are applied e.g. in the electronics industry as substrates for eg. electronic circuits or solar cells.

Another application of the tape casting technique is in the field of fuel cells, more specifically the Solid Oxide Fuel Cell (SOFC) type, where compounds like 8YSZ or 10YSZ (Yttrium Stabilised Zirconium oxide with 8 or 10 mole% of Y2O3) are tape casted to give an electrolyte layer (for electrolyte supported designs) or compounds like La₁₋ₓSrₓMn_{1-y}Co_{y}O₃₋ (LSMC) or La₁₋ₓSrₓFe_{1-y}Co_{y}O₃₋ (LSFC) for anode supported SOFC-designs.

Various production routes for ceramic and metallic foam structures exist. The main characteristics like pore size distribution, relative density, cell morphology and strut density determine the ultimate properties of the foam and thus the field of application. Due to the wide range of application for such materials, several manufacturing routes have been proposed. The major fabrication route is the polyurethane replica technique, where reticulated polyurethane foam is coated with ceramic slurry.

However, the major difficulty with the above mentioned technique exists in a strict control of both the macro- and microstructure of the foam, accompanied with sufficient mechanical strength. For example, the polyurethane replica technique provides foam structures with a well-controlled pore size distribution and strut thickness (owing to a strict process control for the basic material, namely the polyurethane foam). However, as the polymeric foam is calcinated, the strut becomes hollow, giving rise to poor mechanical strength. Other techniques lead to dense struts and strong porous material, but the control over the macro- and microstructure is much more difficult.

As the simultaneous existence of both requirements is essential for the bulk of the applications for ceramic or metallic porous materials, the developments are focussing on new and improved manufacturing routes.

Metallic and ceramic foams are porous materials with very low density (5 to 30% of theoretical density) which are deployed in diverse applications such as filters for molten metals, oven or furnace walls, soot filters, carriers for catalysts, biomedical implants, electro-ceramics, .... Useful implementations of metallic or ceramic foams requires foams with sufficient strength, which have a controlled structure on both micro and macro level.

Several methods for making such metallic or ceramic foams have been described, however none of these methods provide the superior properties needed for some applications.

Methods for making ceramic foams using hollow spheres have been described by D.J. Green, "Fabrication and mechanical properties of lightweight ceramics produced by sintering of hollow spheres", J.Amer.Ceram.Soc. Vol 68,no7, pp 403-409 and by U. Waag et al. in "Metallic hollow spheres - materials for the future", MPR January 2000, pp 29-33.

### Aims of the invention

The present invention aims to provide a new method for producing metallic and ceramic products and in particular for providing a new tape casting method and a new method for producing metallic and ceramic foams. A further aim of the invention is to provide a method for producing strong, closed and/or open metallic and ceramic foams. The new method should provide a precise control on the main characteristics of the product, together with good mechanical strength. Further, the present invention aims at avoiding toxicity and pollution issues as present in the current state of the art methods.

### General Description of the invention

The present invention concerns a method for producing metallic or ceramic products, comprising the steps of:
- Providing a suitable ceramic or metallic slurry,
- Casting said slurry into a predefined form,
- Drying, optionally calcining and sintering said form,
characterised in that said suitable ceramic or metallic slurry comprises a biogel former.

Said biogel former is preferably selected from the group consisting of gelatine, ovalbumin, agar, carageenan, inulin, pectine, starch, potato dextrin, guar, caseinate, gellan, alginate, locust bean gum, xanthan and carboxy-methyl-cellulose. Said casting step is preferably selected from the group consisting of tape casting, hollow shape casting and foam casting.

In a first embodiment, the method of the present invention comprises a method for producing ceramic hollow shapes, comprising the following steps:
- Preparation of a stable ceramic powder slurry comprising a gelling agent, with predefined rheological properties,
- Providing sacrificial support material,
- Coating said support material with said ceramic slurry,
- A drying step, and
- An optional burning step and/or a presintering step depending on the sacrificial support material.

Further, this method can be followed the following steps to obtain a foam structure:
- Arranging hollow shapes as obtained by the method as described higher in a matrix,
- A fixing step wherein said hollow shapes are interconnected by addition of ceramic slurry as above, followed by a drying step.
Preferably, the method further comprising a burning step, a presintering step and a sintering step.

The predefined rheological properties preferably comprise a viscosity lower than 0.01 Pas. The sacrificial support material preferably has dimensions between 20 µm and 5 mm. The method according to this embodiment of the present invention can comprise a burning out step which is advantageously performed at a temperature between 500 and 600°C. The method can also comprise a presintering step which can be performed at a temperature between 1000 and 1200°C.

In the method according to this first embodiment of the present invention, the form factor of the sacrificial support material can selected from the group consisting of spherical, cubical, ellipsoid, cylindrical, tubular, pyramidal and oblong. Any shape is possible.

In a preferable embodiment of the invention, the method further comprises a repetition of the coating steps prior to arranging the hollow shapes in a matrix. This way, the mechanical strength of the hollow shapes and thus also of the foams obtained therewith can be increased. Also preferable is to add a fugitive phase to the stable ceramic powder slurry. This way, an open cell structure can be obtained.

The arranging step can comprise arranging hollow shapes of predetermined dimensions at predetermined positions in said matrix. Also, when hollow shapes of different sizes are arranged to form a gradient, a foam structure gradient is obtained.

In a second embodiment of the present invention, the casting is tape casting. Preferably, the biogel former is gelatine. Further, advantageously the slurry has a rheological behaviour which is shear thinning with viscosity at about η=1 Pa.s at shear rate of 10 s⁻¹. The metallic or ceramic product obtained can have a density which lies between 50 and 100%, preferably between 90 and 100% and advantageously between 95 and 100% theoretical density.

The method of the present invention can also comprise slurry coating of organic polymer foams.

In a third embodiment of the present invention, the ceramic or metallic product is shaped as a foam structure. In this case, the step of providing a suitable ceramic or metallic slurry preferably comprises the steps of:
- Preparation of a stable ceramic or metallic suspension with a suitable dispersant;
- Preparation of a solution of biogel former; and
- mixing the ceramic or metallic suspension with the solution of biogel former.

Preferably, the biogel former is agar. The solid loading of the suspension lies advantageously between 50 and 90 weight%, more advantageously between 60 and 80 weight%.

The method according to this embodiment of the present invention preferably further comprises after the step of casting a gelation step. In a preferred embodiment, the gelation step comprises a temperature change of the slurry to a temperature suitable for gelation of the biogel former.

Another aspect of the present invention is a green ceramic or metallic product obtainable by any of the methods of the present invention and comprising a biogel former. A green ceramic or metallic product is an intermediate product (before calcination and sintering).

### Detailed description of the invention

The present invention concerns the use of natural compounds such as biogel formers (also called hydrocolloid or biobinder) as a binder. Preferably, water-based suspensions are used for this, as the natural compounds can readily dissolve in water. Typical examples of such biogel forming compounds are gelatin, ovalbumin, agar, carageenan, ....

The present invention comprises in a first embodiment a method for producing ceramic hollow spheres and strong, open and/or closed ceramic foams built from said hollow shapes. The metallic and ceramic hollow shapes produced according to the invention preferably have a predetermined size range of 20 µm to 5 mm. The method according to the invention comprises the use of sacrificial support material such as styrene, seeds, nuts, peas,.... The method also comprises the use of a metallic or ceramic slurry, preferably comprising a natural gel-forming compound such as gelatine, agar, carrageen, ....

In a second embodiment, a manufacturing method for making ceramic foams with the aid of biogel formers is described.

The third embodiment of the present invention concerns an environmentally friendly tape casting process for ceramic products with the aid of biogel formers.

### Short description of the figures

Figure 1 a and b show respectively a ceramic foam produced starting from styrene granules and a ceramic foam produced starting from peas and seeds having a different diameter.

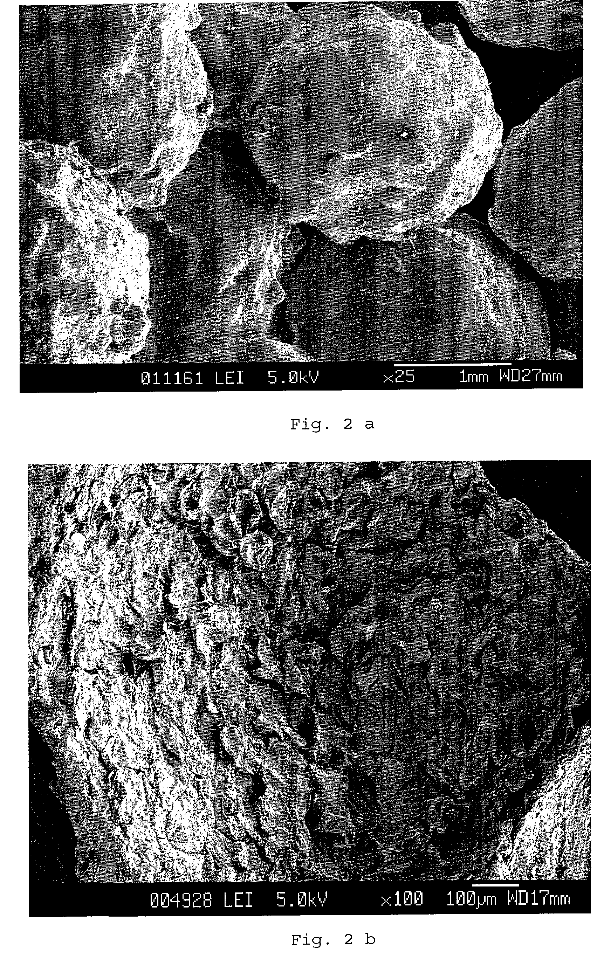

Figure 2 a and b show respectively the structure of the hollow spheres and the surface roughness.

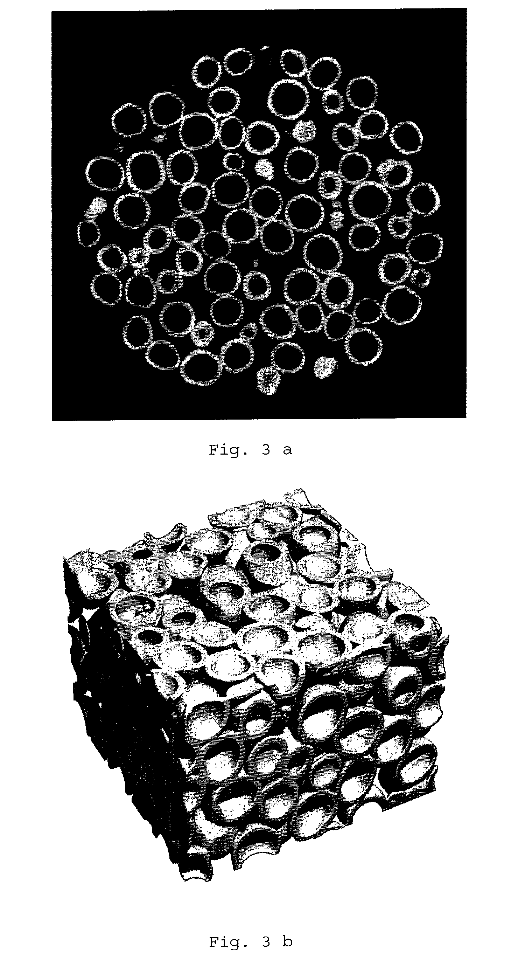

Figure 3 shows an X-ray tomographic analysis of ceramic foam manufactured by the hollow spheres method: (a) two dimensional cross section and (b) multislice reconstruction into three dimensional structure.

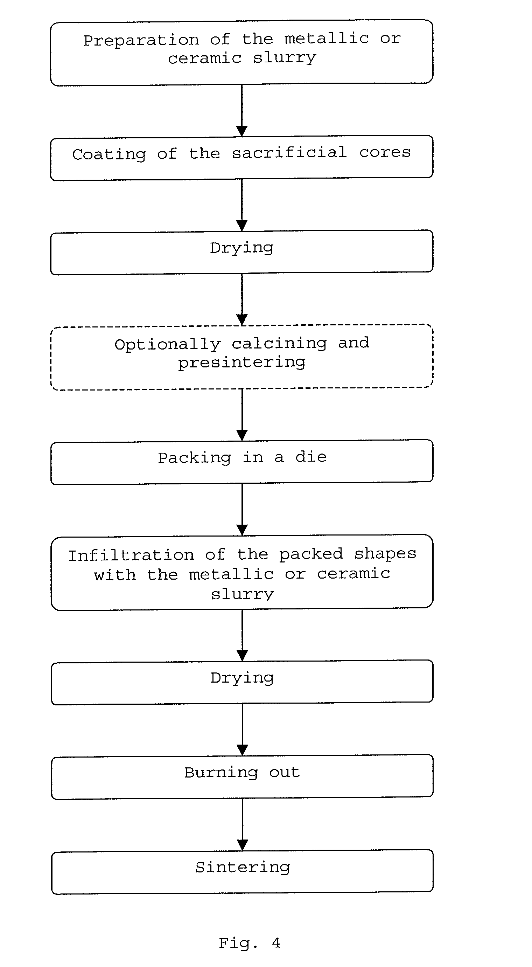

Figure 4 shows a flowchart of the method for producing metallic or ceramic hollow shapes and foams according to the present invention.

Figure 5 shows a flowchart of the method for making ceramic foams with the aid of biogel formers according to the present invention.

Figure 6 shows the influence of (a) the concentration of agar and (b) the concentration of Al₂O₃ on the viscosity of the mixture.

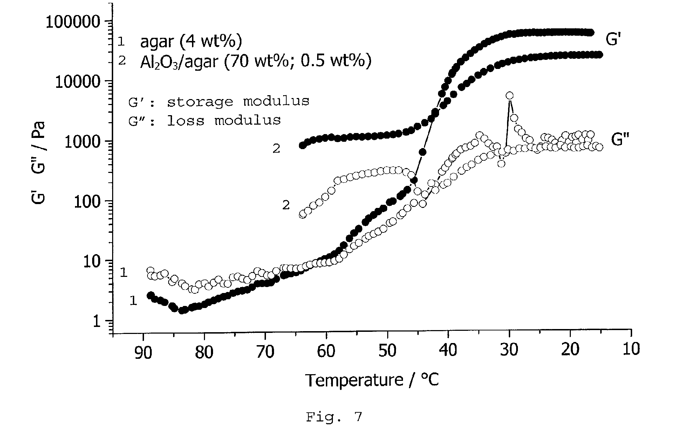

Figure 7 shows the evolution of the viscosity of a solution of agar and a mixture ceramic suspension-agar solution during cooling. The gelation of agar can be observed by the sharp increase of the storage modulus around 40 °C.

Figure 8 depicts the distribution of the cell diameter of a Al₂O₃ porous structure as determined by image analysis (fig. 8 a), together with an optical microscope picture of a 2D slice of the same foam (fig. 8 b).

Figure 9 shows a flowchart of the method for tape casting with the aid of biogel formers according to the present invention.

The method for producing metallic or ceramic hollow shapes more particularly comprises the following steps:
- Preparation of a stable metallic or ceramic powder slurry comprising a gelling agent, with predefined rheological properties (viscosity preferably lower then 0.01 Pas),
- Providing sacrificial support material (such as styrene, seeds, nuts, peas,...),
- Coating said support material with said metallic or ceramic slurry,
- A drying step, and
- An optional burning out step (e.g. 2 hours at a temperature between 500 and 600 °C) depending on the sacrificial support material (if it needs to be burned off).

The metallic or ceramic slurry thus comprises preferably water (as alternative, a solvent can be used), metallic or ceramic powder, dispersant and a bio-gelling agent. Other additives can be added. The coating step can be performed using a spray dry set-up and/or by rolling the support material in a cylinder filled with said metallic or ceramic slurry. The temperature has to be higher than the gelling temperature of the gelling agent. The precise formulation depends on the weight of the support material shapes. The bio-gelling agent provides for wetting the support material surface and by gelling after cooling down for good adhesion.

The method according to the invention for producing metallic or ceramic foams built from hollow shapes further comprises the following steps:
- Arranging hollow shapes as obtained by the method of the invention in a matrix,
- A fixing step wherein said hollow shapes are interconnected by addition of metallic or ceramic slurry as above, followed by a drying step.

The method preferably further comprises a burning out step (500-600°C), and for ceramics a presintering step (1000-1200°C) and a sintering step (1400-1700°C). For metals the sintering temperatures are much lower, starting at about 500°C. The exact temperatures for these different heat treatments depend on the kind of core material and of the metallic or ceramic powder used.

Many uses of the metallic and ceramic materials according to the invention can be imagined. Metallic or ceramic foams created according to the present invention with a tailored cell structure find applications as new thermal isolation material, sensors and actuators, biomaterials and so on.

### Examples

### Example 1

As the sacrificial core, styrene granules with mean size 2 mm are used. The ceramic slurry consists of Al₂O₃ powder, CT3000SG (Alcoa) 15 vol%, a bio-binder e.g. gelatine (1 to 5 wt%/water), water and Darvan C. The viscosity has to be lower than 0.01 Pas

The styrene granules are coated by using a combination of spray drying and turning vessel set up.

The coated styrene granules are packed in a mould and infiltrated with the same ceramic slurry as for the first coating. Hereby the neck formation between the hollow shapes is realised. The heat treatments are as follows:
- Drying at room temperature
- Burning out 2 hours at 600°C
- Presintering 2 hours at 1100°C
- Sintering 1 hour at 1650°C

The compaction strength of the hollow shapes produced from the styrene granules is 22N for a layer thickness of 0.3mm.

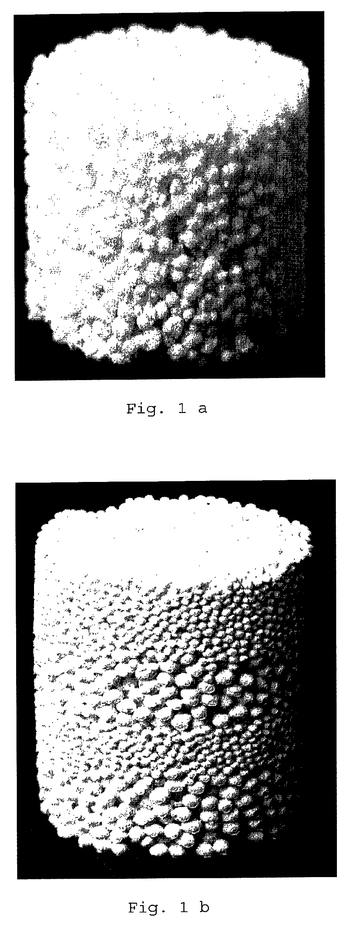

The compaction strength of cylinders built up with these hollow elements is 2.5 MPa for a density of 15-16% (of the theoretical density). The result can be seen in figure 1 a.

### Example 2

- Sacrificial core: peas (diameter 4 mm).
- Slurry idem as in example 1.
- The coating of the peas was done in a turning vessel.
- Packing, second coating and the heat treatment were done as in example 1.

The mechanical compaction strength of the spheres is 22N for a layer thickness of 0.3mm. The mean compaction strength of cylinders built up with this material is 0.9 MPa for a density of 11-12 %.

### Example 3

- Sacrificial core: seed (diameter 2 mm.)
- Slurry idem as in example 1
- Coating, packing, second coating and heat treatment as in example 2

The mean compaction strength of the obtained hollow spheres is 31 N for a layer thickness of 0.3 mm. The mean compaction strength of cylinders built up with these hollow spheres is 4 MPa for a density of 22.5%.

### Example 4

- Coating of seeds and peas is done as in examples 2 and 3.
- Packing is done by providing alternating layers of coated peas and coated seeds.
- Second coating and heat treatment as in example 2.

The result can be seen in figure 1 b. The alternating layers of seeds 1 and peas 2 are clearly visible.

According to the second embodiment of the present invention, a manufacturing method for making ceramic foams with the aid of biogel formers is described.

As important properties of ceramic porous structures like pore size distribution, strut thickness and pore morphology can vary substantially according to the different fabrication routes, these materials are applicable in a wide field. They are used as filters, dust collectors, membranes, catalyst carriers, thermal barriers, lightweight components, bone replacements.... As ceramic foams which are produced with the proposed gel casting procedure using environmentally friendly biogel formers, have greater mechanical strength compared to e.g. the reticulated foam structures with comparable porosity, the applications can even be expanded to situations in which the mechanical strength of the material plays an important role. More specifically, these porous structures could be optimised for their use in the filtration of molten aluminium or other molten metals, as strength and thermal shock resistance are in this application of great importance.

In diesel particulate traps, the characteristics of the ceramic foam must lead to low pressure drop in the filter and at the same time a high trapping efficiency. Accumulated soot can be oxidised using an oxidation catalyst. The ceramic porous structure is in that case used as catalyst support. Mechanical strength is also in this application of great importance.

Several porous structures are already used for bone replacements. A strict control of pore size distribution, porosity and mechanical strength of the foam are essential to guarantee a safe and osteoinductive implant that can either temporarily or life lasting substitute the original bone.

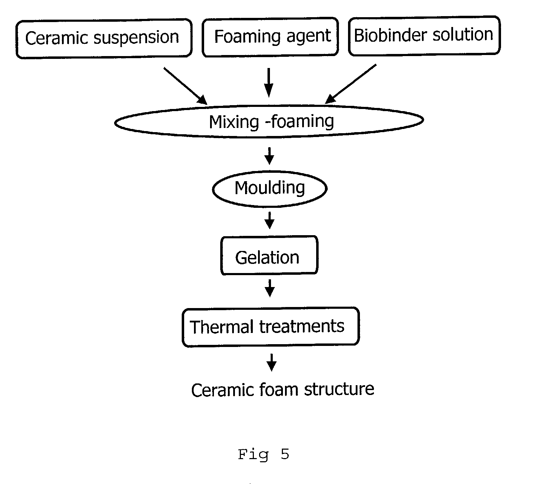

The flow chart of this production method is designed for the production of porous structures with the aid of biogel formers (see fig.5). This manufacturing route makes it feasible to produce ceramic and metallic foams in an environmentally and economically beneficial manner.

Various biogel former systems can be employed in the fabrication of foam structure by gel casting. As naturally occurring gel formers are used as an alternative for the chemical gel formers, major alterations to the conventional gel casting procedure are requisite/inevitable.

The general method can be described as follows:

1. preparation of a stable ceramic suspension, with a suitable dispersant. The solid loading of this suspension can be varied, depending on the desired properties of the foam structure. Preferably, the solid loading is between 60 and 80 weight percentage.

2. preparation of a solution of biogel former. The concentration of this solution can be varied within a specific range (typically preferably between 3 and 7 weight percentage). The experimental conditions are related to the specific biogel former system that will be applied. For example, the use of agar implies heating the mixture above 90 °C. Only in this way, complete dissolution of the agar molecules can be guaranteed. As the gelation takes place at temperatures below 45 °C, this solution must be kept a higher temperature, preventing gelation outside the mould. Alternative systems (based on carrageenan, locust bean gum, xanthan gum, ... or blends of this kind of substances) require other temperatures for handling (dissolution, gelation,...).

3. mixing the ceramic suspension and the biogel former solution. The experimental conditions (like e.g. temperature, other additives) are imposed by the biogel former system used. At this moment, the organic foam agent is added to the mixture. As a result of continuously mixing, a foam structure of ceramic particles in a three dimensional network of biogel former is obtained. The mixing time, the kind of foam agent and the concentration of the foam agent are important experimental parameters as well and contribute to the ultimate foam properties. The mixing time varies preferably between 5 and 10 minutes. During that time, the mixture should be continuously heated. In this way, premature gelation of the biogel former is prevented.

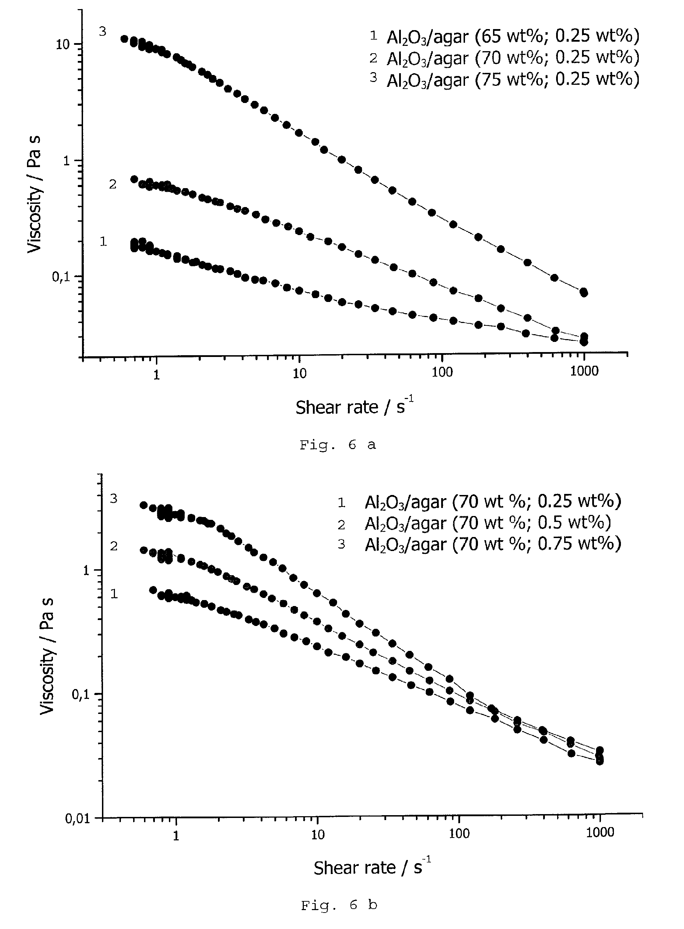

The viscosity of the mixture is determined by both the concentration of ceramic powder and the concentration of biogel former. The mixture ceramic suspension/biogel former solution is characterized by a pseudoplastic behaviour with a viscosity between roughly 10 and 0.1 Pas at = 1 s⁻¹). The influence of both the solid loading and the concentration of agar on the viscosity of the mixture is presented in the figure 6. It can be expected that the viscosity of the mixture plays an important role for the ultimate properties of the foam (cell morphology, stability, homogeneity, ...).

Porosity (or foam) in this mixture of ceramic suspension and biobinder solution can be introduced by several methods, amongst which adding foam agents, followed by mixing and blending in such a way that a homogeneous foam structure is obtained. Another method to create porous structure is the introduction of gas or other substances that release gas at these conditions in the mixture.

The created foam is poured into a mould. The concentration of both ceramic suspension and biogel former solution can be varied en will determine in part some characteristics of the foam.

4. The experimental conditions are changed in such a way gelation of the biogel former occurs. Depending on the type of biogel former, the action to allow gelation may vary. For example, when using agar as a biogel former, the foam structure is allowed to cool in the mould in order to achieve gelation of agar (below 40 °C). The cooling step can proceed at normal speed (at room temperature) or can be accelerated by placing the mould in the refrigerator (at about 4°C) or by freeze-drying.

5. the foam is dried, calcinated (at 500-600°C) and subsequently sintered. The exact temperatures depend on the kind of biogel former and of the metallic or ceramic powders used.

The result of this procedure consists of a ceramic foam structure, of which the properties like porosity (5 to 30 %), pore size distribution and strut thickness are fully controlled. These properties can be changed within certain limits, depending on the interaction between several experimental conditions. Moreover, the mechanical strength of such porous structures is high. Unlike foam structures manufactured by the polyurethane replica technique, the struts of gelcasted ceramic foams are dense. The procedure involves environmentally friendly and relatively cheap starting products. The figure 5 gives a schematic overview of the procedure.

### Examples

### Example 5: mechanical strength of the ceramic foam

A 7 wt% aqueous agar solution is heated to 100 °C in order to assure complete dissolution of the agar molecules. The ceramic suspension consists of 66 wt% Al₂O₃ with Darvan C as a dispersant (1.4 ml/100 g ceramic powder). Before mixing the solutions, the ceramic suspension is ball milled for 30 min. During mixing, the gelation of the agar is prevented by continuously heating the mixture. This mixture consists of 48.7 wt% Al₂O₃ and only 1.7 wt% agar. A foaming agent is added (Tergitol TMN10, Fluka Chemika) and the mixture is stirred for 5 min. The resulting foam structure is allowed to cool in a mould. After drying, calcination and sintering, the ceramic foam structure is cut into shapes. The porosity of this structure has been determined as 85 %. The mechanical strength has been tested by 3-point bending tests. The mean bending strength for this particular foam structure is 3.8 MPa. For comparison, foam structures with comparable porosity and which are manufactured by the replica technique are characterised by bending strength not exceeding 2 MPa.

### Example 6: Cell diameter

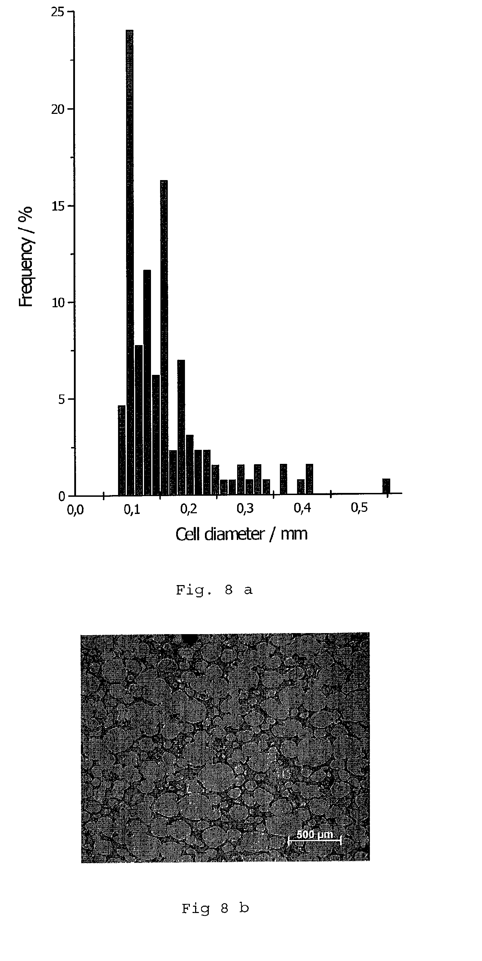

Depending on the different experimental parameters like solids loading, nature and concentration of the foaming agent, mixing time and agar concentration, a variety of foam structures can be obtained each with their characteristic distribution of cell diameters. As an example, a typical cell diameter distribution is presented in figure 8 a, together with a optical microscope picture of an imbedded slice of the foam (figure 8 b).

For the production of this structure, a 50 wt% Al₂O₃-suspension (with Darvan C as dispersant) was heated to 60 °C and mixed with a 4 wt% agar solution that had been heated at 90 °C to assure complete dissolution. After adding a foaming agent (Tergitol TMN10, Fluka Chemika), the mixture is mixed using a conventional mixer for 2 min. The composition of the final mixture consists of 44 wt% Al₂O, 1.8 wt% agar and 0.9 vol/wt% foaming agent. The gelation of the biobinder is accelerated by cooling in the refrigerator for 1 h. Afterwards, the ceramic structure is allowed to dry at room temperature, is calcinated (600°C) and sintered at 1700 °C.

The third embodiment of the present invention concerns an environmentally friendly tape casting process for ceramic products.

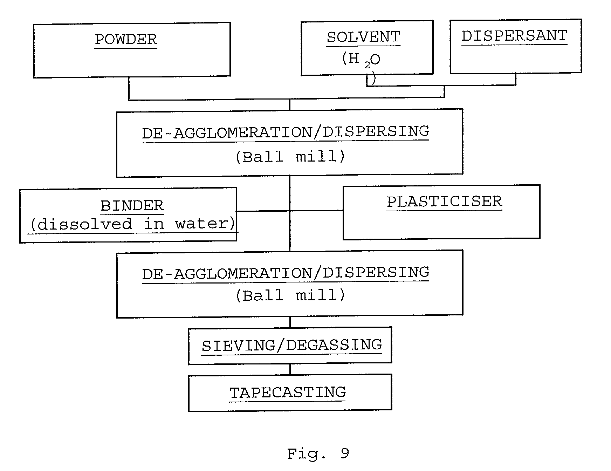

In the flowchart as seen in figure 9, the method to prepare a water based tape casting suspension with hydrocolloid is outlined.

### Examples:

In the tables below, typical suspension compositions are presented:

### Example 7:

**Table 1:**

| composition 1 | | |
|---|---|---|
| **Function** | **constituent** | **Amount (wt%)** |
| Ceramic powder | Al₂O₃ (AKP30) | 57.1 |
| Solvent | water | 39.09 |
| Dispersant | Darvan C | 1.43 |
| Binder | gelatine | 1.43 |
| Plasticiser | Polyethylene glycol | 1.15 |

### Example 8:

**Table 2:**

| composition 2 | | |
|---|---|---|
| **Function** | **constituent** | **Amount (wt%)** |
| Ceramic powder | 8YSZ | 58.4 |
| Solvent | Water | 36.7 |
| Dispersant | Darvan C | 1.66 |
| Dispersant/plasticiser | Linseed oil | 0.44 |
| Binder | Gelatine | 2.38 |
| Plasticiser | Polyethylene glycol | 0.19 |
| Wetting agent | FC-431 | 0.39 |

The composition is chosen for reasons of optimal rheology (to fit the tape cast process) and stability. The solid loading is preferably as high as possible for the concentration of organic constituents to be kept as low as possible. A too high solid content gives a suspension too thick to cast, which cannot be removed from the milling balls, and which cannot easily be de-aired. A lower solid content gives a suspension too thin for good casting results. The plasticiser is added for tape flexibility: too little plasticiser gives brittle tape that cannot be handled without causing breaking or cracks.

For tape casting, the following procedure can be followed:
- preparation of a stable ceramic powder suspension, comprising a ceramic powder, a dispersing medium, a dispersant and a binder. Optionally, wetting agent, surfactant, plasticiser or anti-foaming agents are added. The rheology of the suspension is controlled to allow casting at suitable speeds and width gap (see below). The rheological behaviour of the suspension is shear thinning with typical viscosity η=1 Pa.s at shear rate of 10 s⁻¹.
- In order to remove the milling balls from the suspension the content of the ball mill is poured over a coarse sieve.
- Degassing is done in order to remove entrapped air bubbles from the suspension. Air bubbles in the sintered tape can be seen as flaws that reduce the mechanical properties of the tape.
- Tape casting of the ceramic material: on mylar or silicone coated mylar as a substrate film, cast speeds typically between 0.25 cm/s to 5 cm/s, gap between the doctor blade and the substrate film typically 100 to 500 micron, temperature of the suspension at casting: usually room temperature or above (in function of rheology).
- A drying step at room temperature.
- A calcining step at 500-600°C and a sintering step. The exact temperatures for these different heat treatments depend on the kind of biogel former and binders and of the metallic or ceramic powders used.

Tapes were casted with the above-mentioned method. Densities obtained were as high as 95 to even 98 % theoretical density, making this technique suitable for e.g. making gas-tight membranes.

## Claims

1. A method for producing metallic or ceramic products, comprising the steps of:
• Providing a suitable ceramic or metallic slurry,
• Casting said slurry into a predefined form,
• Drying, optionally calcining and sintering said form,
**characterised in that** said suitable ceramic or metallic slurry comprises a biogel former.

2. Method as in claim 1, **characterised in that** said biogel former is selected from the group consisting of gelatine, ovalbumin, agar, carageenan, inulin, pectine, starch, potato dextrin, guar, caseinate, gellan, alginate, locust bean gum, xanthan, carboxy-methyl-cellulose.

3. Method as in claim 1 or 2, **characterised in that** said casting is selected from the group consisting of tape casting, hollow shape casting, foam casting.

4. A method for producing ceramic hollow shapes, comprising the following steps:
• Preparation of a stable ceramic powder slurry comprising a gelling agent, with predefined rheological properties,
• Providing sacrificial support material,
• Coating said support material with said ceramic slurry,
• A drying step, and
• An optional burning step and/or a presintering step depending on the sacrificial support material.

5. A method for producing ceramic foams built from hollow shapes comprising the following steps:
• Arranging hollow shapes as obtained by the method of claim 4 in a matrix,
• A fixing step wherein said hollow shapes are interconnected by addition of ceramic slurry as above, followed by a drying step.

6. The method as in claim 5, further comprising a burning step, a presintering step and a sintering step.

7. The method as in any of the claims 4 to 6, wherein the sacrificial support material has dimensions between 20 µm and 5 mm.

8. The method as in any of the claims 4 to 7, wherein the form factor of the sacrificial support material is selected from the group consisting of spherical, cubical, ellipsoid, cylindrical, tubular, pyramidal and oblong.

9. The method of any of the claims 5 to 8, further comprising a repetition of the steps of claim 4 prior to any of the steps of claim 5.

10. The method as in any of the claims 4 to 9, wherein the stable ceramic powder slurry further comprises a fugitive phase.

11. The method as in any of the claims 5 to 10, wherein the arranging step comprises arranging hollow shapes of predetermined dimensions at predetermined positions in said matrix.

12. The method as in claim 11, wherein hollow shapes of different sizes are arranged to form a gradient.

13. The method as in claim 1 or claim 2, wherein the casting is tape casting.

14. The method as in claim 13, wherein the biogel former is gelatine.

15. The method as in claim 13 or 14, wherein the metallic or ceramic product obtained has a density which lies between 50 and 100%, preferably between 90 and 100%, advantageously between 95 and 100% theoretical density.

16. The method as in claim 1 or claim 2, comprising slurry coating of organic polymer foams.

17. The method as in claim 1 or claim 2, wherein the ceramic or metallic product is shaped as a foam structure.

18. The method as in claim 17, wherein the step of providing a suitable ceramic or metallic slurry comprises the steps of:
• Preparation of a stable ceramic or metallic suspension with a suitable dispersant;
• Preparation of a solution of biogel former; and
• mixing the ceramic or metallic suspension with the solution of biogel former.

19. The method as in claim 17 or 18, wherein the biogel former is agar.

20. The method as in claim 18, wherein the solid loading of the suspension lies between 50 and 90 weight%.

21. The method as in any of the claims 17 to 20, further comprising after the step of casting a gelation step.

22. The method as in claim 21 wherein the gelation step comprises a temperature change of the slurry to a temperature suitable for gelation of the biogel former.

23. Green ceramic or metallic product obtainable by the method as in any of the claims 1 to 22 and comprising a biogel former.
